# EUROPEAN PATENT APPLICATION

(11) **EP 4 599 792 A1**
(43) Date of publication of application: **13.08.2025**
(21) Application number: 23875041.8
(22) Date of filing: 21.08.2023
(51) Int. Cl.: A61C 7/00, A61C 9/00, A61B 5/00, A61B 6/03, G16H 50/50, G16H 30/00, A61C 1/00, G06T 7/00, A61B 18/20

(54) **METHOD FOR GENERATING ORTHODONTIC TREATMENT PLAN, AND DEVICE THEREFOR**

(30) Priority: 06.10.2022 KR 20220128100
(71) Applicant: Osstem Implant Co., Ltd., Gangseo-gu Seoul 07789 (KR)
(72) Inventor: CHOI, Kyoo Ok, Seoul 07789 (KR); KIM, Hwa Sam, Gimpo-si Gyeonggi-do 10113 (KR)
(74) Representative: Klunker IP Patentanwälte PartG mbB
(86) International application number: PCT/KR2023/012347
(87) International publication number: WO 2024/075971

(57) **Abstract**

Provided are a method for generating an orthodontic treatment plan and a device therefor, wherein an analysis result of one or more pieces of scan data for a patient is used to generate element data needed for orthodontic treatment from the scan data, problem items, identified using the element data, for the orthodontic treatment of the patient are displayed, and information, generated using the identified problem items, about an orthodontic treatment goal for the patient is displayed. Additionally, provided are a method for generating an orthodontic treatment plan and a device therefor, wherein an orthodontic treatment plan, generated using the generated information about the orthodontic treatment goal, for the patient is displayed, and a control signal for manufacturing an orthodontic device used for the orthodontic treatment of the patient is output using the generated orthodontic treatment plan.

## Description

### [Technical Field]

The present disclosure relates to a method for generating an orthodontic treatment plan. More specifically, the present disclosure relates to a method for generating an orthodontic treatment plan and a device therefor, which minimizes user intervention during the orthodontic treatment process.

### [Background Art]

A method and device for generating an orthodontic treatment plan are provided. Users may establish a dental orthodontic treatment plan for patients based on digital data as well as the users' knowledge and experience. In this process, system operations such as data input and modifications by the user are involved during the diagnosis and analysis of the patient's issues, presentation of treatment objectives, and establishment of the treatment plan.

Accordingly, there is a need for technology that enhances user convenience, as well as the speed and accuracy of orthodontic treatment, by minimizing user intervention during the process of generating an orthodontic treatment plan.

### [Disclosure]

### [Technical problem]

A technical objective of the present disclosure is to provide a method and device for generating an orthodontic treatment plan that minimizes user intervention.

Another technical objective of the present disclosure is to provide a method and device for generating an orthodontic treatment plan, which enables the process of generating an orthodontic treatment plan to be performed in an automated manner.

Another technical objective of the present disclosure is to provide a method and device for generating an orthodontic treatment plan, which reduces the working time and delivers the required level of completeness regardless of the user's level of expertise.

Objectives of the present disclosure are not limited to the above-mentioned objectives, and other unmentioned objectives should be clearly understood by those of ordinary skill in the art to which the present disclosure pertains from the description below.

### [Technical Solution]

According to one embodiment of the present disclosure, a method for generating an orthodontic treatment plan may include analyzing one or more scan data for a patient and generating element data necessary for orthodontic treatment from the one or more scan data by using the analysis result, identifying a problem item for the orthodontic treatment of the patient using the element data and displaying the identified problem items, and generating an orthodontic treatment objective for the patient using the identified problem item and displaying information for the generated orthodontic treatment objective.

In one embodiment, the scan data may be data acquired using an intraoral scanner or a skeletal computed tomography (CT), and the element data may be data obtained by preprocessing the scan data into a format required for identifying a problem item.

In one embodiment, the generating of the element data may include generating element data by matching multiple scan data among the one or more scan data.

In one embodiment, the generating of the element data may include generating the element data by separating the one or more scan data.

In one embodiment, the displaying of the problem item may include determining a position and a direction in which the problem item is to be displayed and displaying the problem item according to the determined position and direction.

In one embodiment, the displaying of the problem item may include, when multiple data related to the problem item are displayed in an overlapping manner, displaying the data by distinguishing each data using identifiable visual information.

In one embodiment, the displaying of the information for the orthodontic treatment objective may include generating the orthodontic treatment objective for the patient using the identified problem item. In this case, the orthodontic treatment objective may be generated in a manner that reduces deviation from a reference value of the identified problem item.

In one embodiment, the displaying of the information for the orthodontic treatment objective may include updating a screen displaying the identified problem item to display the information for the orthodontic treatment objective, along with an analysis numerical value adjusted based on the orthodontic treatment objective.

In one embodiment, the displaying of the information for the orthodontic treatment objective may include, when multiple data related to the information for the orthodontic treatment objective are displayed in an overlapping manner, displaying the data by distinguishing each data using identifiable visual information.

According to another embodiment of the present disclosure, a method for generating an orthodontic treatment plan may further include generating an orthodontic treatment plan for the patient using the information for the generated orthodontic treatment objective and displaying the generated orthodontic treatment plan, and outputting a control signal for fabricating an orthodontic device used for orthodontic treatment of the patient, based on the generated orthodontic treatment plan.

In one embodiment, the outputting of the control signal may include converting the orthodontic treatment plan into a medical chart format, displaying the orthodontic treatment plan converted into the medical chart format, and transmitting the orthodontic treatment plan converted into the medical chart format to a hospital's patient management system (PMS) so that the converted orthodontic treatment plan is recorded in an electronic chart of the patient.

According to one embodiment of the present disclosure, a device for generating an orthodontic treatment plan may include analyzing one or more scan data for a patient and generating element data necessary for orthodontic treatment from the one or more scan data by using the analysis result, identifying problem items for the orthodontic treatment of the patient using the element data and displaying the identified problem items, and generating an orthodontic treatment objective for the patient using the identified problem item and displaying information for the generated orthodontic treatment objective.

According to another embodiment of the present disclosure, a device for generating an orthodontic treatment plan may further include generating an orthodontic treatment plan for the patient using the information for the generated orthodontic treatment objective and displaying the generated orthodontic treatment plan, and outputting a control signal for fabricating an orthodontic device used for orthodontic treatment of the patient, based on the generated orthodontic treatment plan.

### [Effects of the Invention]

According to the embodiment, convenience, speed, accuracy, and precision can be enhanced during the generation of an orthodontic treatment plan.

According to the present embodiment, the reproducibility of issues can be improved by utilizing a variety of associated digital data, including information on the patient's soft and hard tissues.

According to the embodiment, a system first presents results at each stage of orthodontic treatment plan generation, so that the frequency and duration of user intervention can be reduced.

According to the embodiment, the orthodontic treatment plan is generated without relying on the user's expertise, thereby minimizing errors and deviations in the orthodontic treatment plan and improving the accuracy and precision of the orthodontic treatment.

According to the embodiment, results presented first by the system at each stage of treatment plan generation are displayed on a single screen, enabling users to easily understand information associated with the orthodontic treatment.

### [Description of Drawings]

FIG. 1 is a conceptual diagram illustrating the overall flow of a method for generating an orthodontic treatment plan according to one embodiment of the present disclosure.
FIG. 2 is a flowchart illustrating a method for generating an orthodontic treatment plan according to one embodiment of the present disclosure.
FIGS. 3 to 5 are example diagrams of a screen for setting a reference value according to one embodiment of the present disclosure.
FIG. 6 is an example diagram of a screen initially displayed in step S100 according to one embodiment of the present disclosure.
FIG. 7 is an exemplary flowchart for describing detailed processes of some operations shown in FIG. 2.
FIG. 8 is an exemplary flowchart for describing detailed processes of some operations shown in FIG. 7.
FIG. 9 is an example diagram illustrating a screen for matching multiple data in step S100 according to one embodiment of the present disclosure.
FIG. 10 is an example diagram of a screen for data separation in step S100 according to one embodiment of the present disclosure.
FIG. 11 is an exemplary flowchart for describing detailed processes of some operations shown in FIG. 2.
FIG. 12 is an example diagram of a problem item display screen in step S200 according to one embodiment of the present disclosure.
FIG. 13 is an example diagram of a detailed analysis result screen for problem items in step S200 according to one embodiment of the present disclosure.
FIG. 14 is an example diagram of a screen for adjusting the range of analysis values for problem items in step S200 according to one embodiment of the present disclosure.
FIG. 15 is an example diagram of a screen in which the detailed analysis results for the problem items in step S200 are classified according to one embodiment of the present disclosure.
FIG. 16 is an exemplary diagram of a screen displaying individual tooth measurement information in step S200 according to one embodiment of the present disclosure.
FIG. 17 is an exemplary flowchart for describing detailed processes of some operations shown in FIG. 2.
FIG. 18 is an example diagram of a screen displaying orthodontic treatment objective results in step S300 according to one embodiment of the present disclosure.
FIG. 19 is an example diagram of an orthodontic treatment objective comparison screen in step S300 according to one embodiment of the present disclosure.
FIG. 20 is an example diagram of a screen for modifying tooth arrangement in step S300 according to one embodiment of the present disclosure.
FIG. 21 is an example diagram of a summary screen of the orthodontic treatment objectives in step S300 according to one embodiment of the present disclosure.
FIG. 22 is an example diagram of a screen showing individual tooth measurement for the orthodontic treatment objectives in step S300 according to one embodiment of the present disclosure.
FIG. 23 is a flowchart illustrating a method for generating an orthodontic treatment plan according to another embodiment of the present disclosure.
FIG. 24 is an exemplary flowchart for describing detailed processes of some operations shown in FIG. 23.
FIG. 25 is an example diagram of a screen showing the results of a treatment process in step S400 according to one embodiment of the present disclosure.
FIG. 26 is an example diagram of a screen showing step-by-step details of the treatment process in step S400 according to one embodiment of the present disclosure.
FIG. 27 is an example diagram of a screen displaying improvements in analysis items based on tooth movement changes in step S400 according to one embodiment of the present disclosure.
FIG. 28 is an example diagram of a screen displayed when additional fixation force is required due to tooth movement in step S400 according to one embodiment of the present disclosure.
FIG. 29 is an exemplary flowchart for describing detailed processes of some operations shown in FIG. 23.
FIG. 30 is an example diagram of a screen displaying treatment device data for certain steps included in the treatment plan finalized through step S400 according to one embodiment of the present disclosure.
FIG. 31 is an example diagram of a screen displaying information in a format suitable for inclusion in a clinical chart in step S500 according to one embodiment of the present disclosure.
FIG. 32 is an example diagram of a screen displaying a document-printable format for step S500 according to one embodiment of the present disclosure.
FIG. 33 is a hardware configuration diagram of a computing device according to some embodiments of the present disclosure.

### [Mode of the Invention]

Hereinafter, example embodiments of the present disclosure will be described in detail with reference to the accompanying drawings. The advantages and features of the present disclosure and the manner of achieving the advantages and features will become apparent with reference to embodiments described in detail below together with the accompanying drawings. However, the present disclosure may be implemented in many different forms and should not be construed as being limited to the embodiments set forth herein, and the embodiments are provided such that this disclosure will be thorough and complete and will fully convey the scope of the present disclosure to those skilled in the art, and the present disclosure is defined only by the scope of the appended claims.

In describing the present disclosure, if it is determined that a detailed description of a related known configuration or function may obscure the gist of the present invention, the detailed description will be omitted.

Hereinafter, some embodiments of the present disclosure will be described with reference to the accompanying drawings.

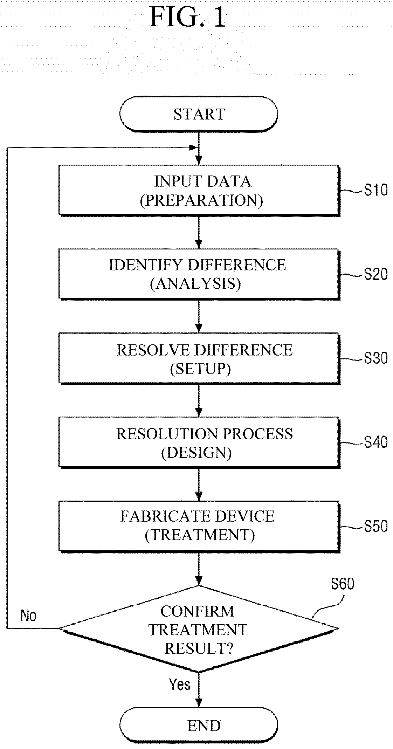

FIG. 1 is a conceptual diagram illustrating the overall flow of a method for generating an orthodontic treatment plan according to one embodiment of the present disclosure.

Referring to FIG. 1, a method for generating an orthodontic treatment plan performed by a computing device may be divided into step S10 of receiving data from a user, step S20 of identifying the difference between a value derived for each analysis item based on the received data and a reference value preset by the user, step S30 of resolving the difference, step S40 of generating a resolution process, and step S50 of fabricating a device.

In step S10, the computing device may receive various data required for the patient's orthodontic treatment from the user, such as intraoral data, skeletal data, and facial data of the patient. Additionally, the computing device may perform a process to align three-dimensional (3D) positions and sizes of the input data received in step S10 to enable the combined use of two or more types of data. At this time, the computing device may adjust the position and size of the data based on intraoral scanner data or skeletal CT data containing accurate 3D size data.

Furthermore, the computing device may perform a tooth segmentation process to distinguish individual teeth and gum data included in the intraoral data, and may display the results of the segmentation in different colors for each individual tooth and the gum, allowing the user to easily assess the segmented data.

Meanwhile, the segmented individual tooth may be reproduced as a realistic tooth shape, including a root connected to the tooth, when skeletal CT data is utilized. The individual tooth may be represented with axis data (e.g., long axis and crown axis) and feature points (e.g., adjacent points and occlusal points). Additionally, the skeletal and facial data are characterized by displaying anatomical feature points.

In step S20, the computing device may calculate the relationships, such as distances and angles, between the anatomical feature points of skeletal and facial data and axis feature points of individual tooth data for each analysis item. Additionally, the computing device may display the difference between the value derived for each analysis item based on the input data provided by the user and the reference value. In this case, the reference value may be preset by the user. More specifically, the reference value may be set according to the user's clinical judgment for each analysis item or based on the patient's preferences. Furthermore, by reflecting the connections between the analysis items, related items may be displayed overlapping, or simultaneously, on a screen, enabling the user to easily verify the derived results.

In step S30, the computing device may set one or more analysis items with differences between the derived values and the reference values in step S20 to be problem items and display hard tissue improvements to resolve these problem items. During the process in which the computing device derives these improvements, tooth arrangement, occlusal relationships, space acquisition plans, and the like, which can reflect the preset reference values, may be sequentially processed. Furthermore, orthodontic treatment objectives incorporating the hard tissue improvements may be finalized through a target modification process for the problem items performed by the user or a modification process for hard tissue data performed by the computing device. At this time, displacement resulting from hard tissue changes targeted by the orthodontic treatment objectives, an overlay of the current state and the orthodontic treatment objectives, and associated soft tissue changes may be displayed together.

In step S40, the computing device may present the process required to achieve the orthodontic treatment objective. During the process in which the computing device derives this process, procedures to avoid collisions between hard tissues during the progressive movement of the hard tissue, which follows the same method as the orthodontic treatment, may be derived. At this time, any necessary devices for the derived process may also be displayed, and the problem-item resolving process reflecting the displacement caused during the treatment process may be displayed.

In step S50, the computing device may output the data finalized through step S40 using a 3D printer to produce a device used for orthodontic treatment. At this time, treatment plan information that can be utilized during the orthodontic treatment process may be presented in text form and may also be linked to a charting program that records the treatment process.

Then, in step S60, the computing device may confirm the orthodontic (treatment) results during the actual orthodontic treatment of the patient using the orthodontic device output in step S50 or after the treatment is completed. Specifically, during the process of wearing orthodontic devices at each step, if information on the patient's dental condition before wearing the orthodontic device for step N+1 is provided, the computing device may compare this information with the orthodontic treatment objective targeted by the orthodontic device at step N to confirm the treatment results. Alternatively, if information for the patient's dental condition is input after all planned orthodontic devices have been worn, the computing device may compare the input information with the final target orthodontic treatment objectives to confirm the treatment results. Here, the information for the patient's dental condition may include not only one or more scan data but also a clinical record based on the user's visual inspection.

Subsequently, if the computing device identifies discrepancies between the input information for the patient's dental condition and the corresponding orthodontic state, the computing device may revert to step S10 and regenerate the orthodontic treatment plan.

According to the present disclosure, the computing device may utilize the patient's hard and soft tissue data obtained during the long-term orthodontic treatment process to compare against the orthodontic treatment results, and in the event of discrepancies with the orthodontic treatment plan, the computing device may generate a procedure to correct these discrepancies and establish a new treatment plan.

Accordingly, a system may first present the results derived throughout the entire orthodontic treatment process, allowing the user to review the presented results, thereby ensuring consistent results from the orthodontic treatment plan.

**In** addition, as the system displays the results derived at each step of the orthodontic treatment process in advance, the speed, convenience, accuracy, and precision of the orthodontic treatment process may be enhanced, and furthermore, key elements of the orthodontic treatment process are displayed on the screen to allow the user to easily identify, thereby improving usability. Moreover, if modifications to the orthodontic treatment process are required due to an additional user request, a structure where the user can make changes while reviewing the basis for the results presented by the system.

The screens presented below are illustrative examples, and the positions of the screens or types of data are not limited to the examples shown. **In** other words, further improvements or changes to the screens may be made based on user needs.

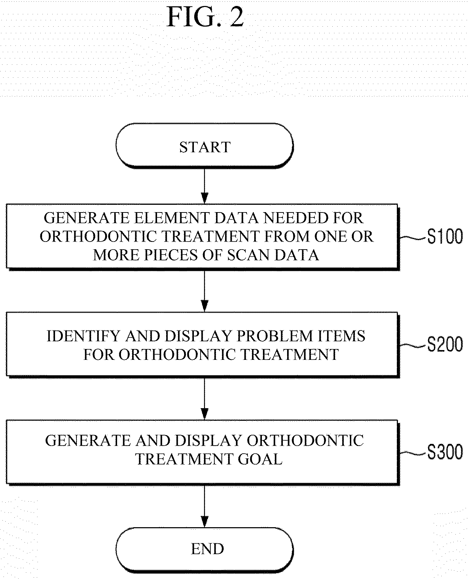

Hereinafter, a method for generating an orthodontic treatment plan according to the present disclosure will be described in more detail with reference to FIGS. 2 to 32.

FIG. 2 is a flowchart illustrating a method for generating an orthodontic treatment plan according to one embodiment of the present disclosure. However, this is a mere exemplary embodiment for achieving the objectives of the present disclosure, and some steps may be added or omitted, as necessary.

As shown in FIG. 2, the method for generating an orthodontic treatment plan may begin with step S100 where a computing device generates element data required for orthodontic treatment from one or more scan data of a patient.

More specifically, in step S100, the computing device may analyze one or more scan data of the patient and generate element data necessary for orthodontic treatment using the analysis results. At this time, the one or more scan data may be data acquired using an intraoral scanner or skeletal CT, and the element data may be data obtained by preprocessing the scan data into a format required for identifying problem items.

In step S200, the computing device may identify and display problem items for orthodontic treatment. More specifically, the computing device may use the element data generated in step S100 to identify problem items for orthodontic treatment for the patient and display the identified problem items.

In step S300, the computing device may generate and display orthodontic treatment objectives. More specifically, the computing device may generate orthodontic treatment objectives for the patient using the problem items identified in step S200 and display information for the generated orthodontic treatment objective.

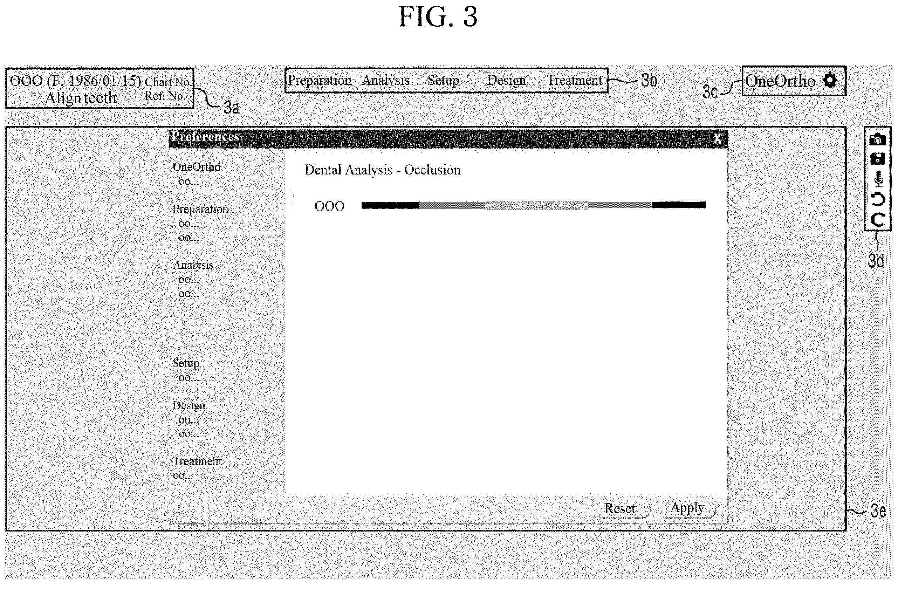

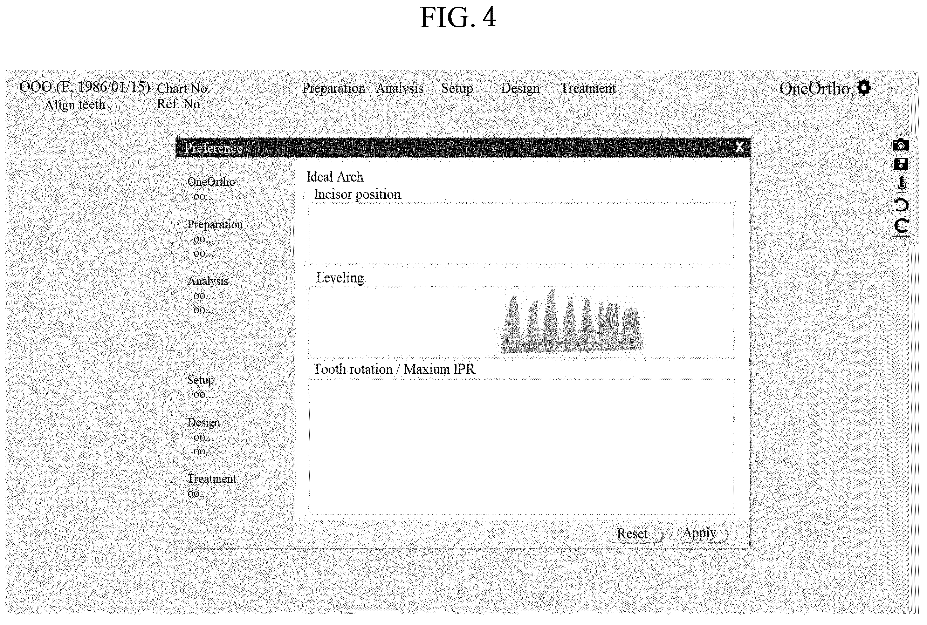

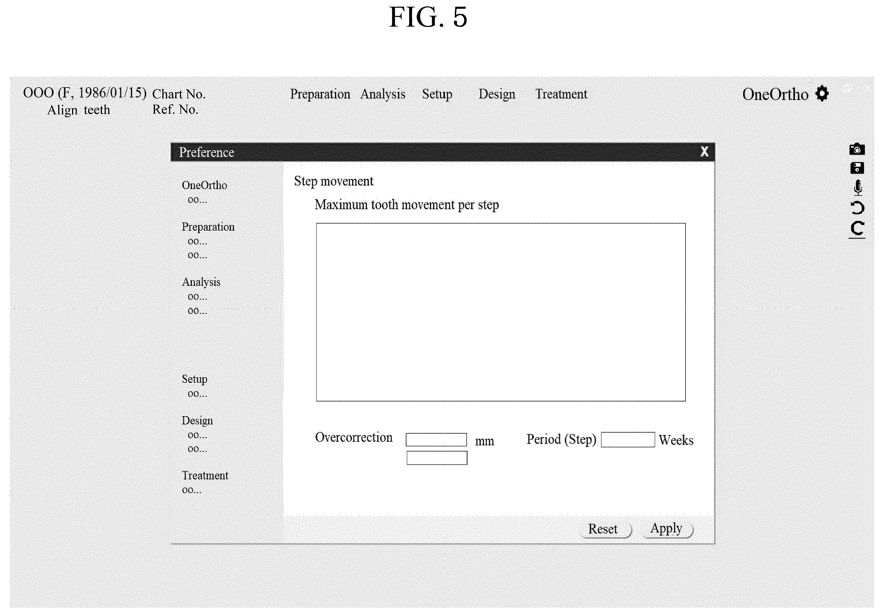

FIGS. 3 to 5 are example diagrams of a screen for setting a reference value according to one embodiment of the present disclosure. FIG. 3 is an example diagram of a screen for setting a reference value associated with step S100, FIG. 4 is an example diagram of a screen for setting a reference value associated with step S200, and FIG. 5 is an example diagram for setting a reference value associated with a detailed orthodontic treatment plan.

As shown in FIG. 3, a displayed screen 300 may be divided into the following areas: an area 3a displaying patient information, medical chart information, management number, and patient request information; an area 3b displaying each stage of the orthodontic treatment plan; an area 3c allowing the selection to navigate to a user settings screen; an area 3d displaying orthodontic treatment plan tools; and an area 3e displaying related content accompanying the orthodontic treatment plan process.

Referring to FIG. 3, the area 3d displaying the orthodontic treatment plan tools may include icons such as a camera icon, a save icon, a recording icon, and icons for navigating to the previous or next screens. More specifically, in response to user input on the camera icon, all or part of the currently displayed screen may be captured; in response to user input on the save icon, data displayed on the current screen may be saved; and in response to user input on the recording icon, conversations between the user and the patient may be recorded.

However, the icons displayed in the area 3d for the tools for the orthodontic treatment plan are not limited to the examples illustrated in FIG. 3, and multiple icons with various functions, such as an icon for converting a graphically displayed item into a numerical value or an icon for documenting and displaying the content output on the current screen, may be additionally displayed as needed by the user.

Referring to FIGS. 3 to 5, the user may predefine the reference values necessary for deriving problem items, resolving problem items, and planning solutions for those problems. Accordingly, a consistent treatment plan may be established by utilizing the reference values for each item that are input in advance by the user. Meanwhile, the user's clinical knowledge may be incorporated in advance through the process of modifying or changing the problem items for each item. In addition, the problem items may be freely modified by the user as needed at any desired time.

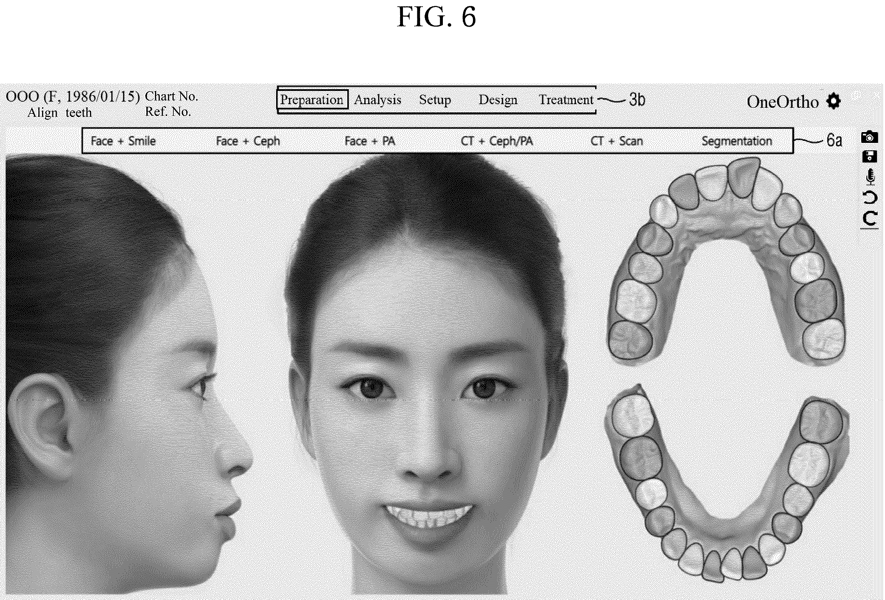

FIG. 6 is an example diagram of a screen initially displayed in step S100 according to one embodiment of the present disclosure.

As shown in FIG. 6, related content associated with the orthodontic treatment plan process may be divided into multiple items as needed. At this time, area 6a, where the multiple items are arranged in a single row, may be displayed at the top portion of the area 3e, which displays the related content associated with the orthodontic treatment plan process.

Referring to FIG. 6, in the area 6a where the multiple items are arranged in a single row may display items corresponding to each combination of various types of data input in step S100. The data used in these combinations may include data for facial scan or photographic image (Face), data for a smiling face (Smile), cephalometric imaging data (Ceph), X-ray imaging data (PA), computed tomography image data (CT), intraoral scan image data (Scan), and segmented individual tooth data (Segmentation). At this time, the data for the smiling face (Smile) represents data on the number of visible teeth, tooth shapes, and gum contours as seen in a smiling face, but it may also include ideal smile data, which is information about the number of visible teeth, tooth shapes, or gum contours that can be optimally displayed in a smiling face. This ideal smile data may be used in a subsequent stage, such as an analysis stage, a setup stage, or the like.

For example, in response to user input on item "Face + Smile" in the area 6a where multiple items are arranged in a single row as shown in FIG. 6, the computing device may combine facial scan or photographic image data with data on the number of teeth, shapes, and gum contours visible when smiling and display the combined data in the area 3e, where related content associated with the orthodontic treatment plan process is displayed.

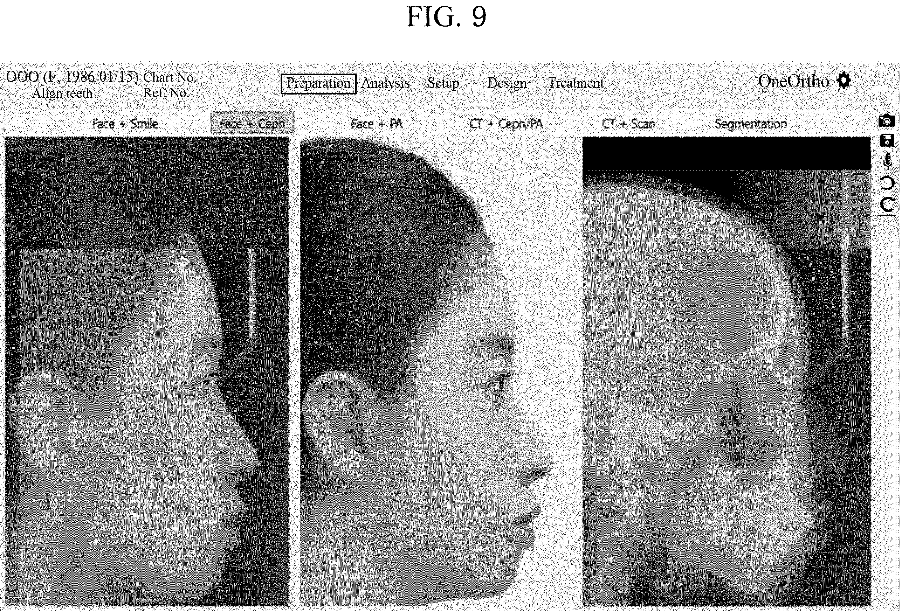

Similarly, as shown in FIG. 9, in response to user input on item "Face + Ceph" in the area 6a where multiple items are arranged in a single row, the computing device may combine the facial scan or photographic image data with cephalometric imaging data and display the combined data in the area 3e, where related content associated with the orthodontic treatment plan process is displayed.

Meanwhile, an item corresponding to a combination including such data may be selected only when data for the user exists, and the computing device may display the result of matching data for the corresponding combination on the screen. In other words, if there is no data for the user, the item corresponding to a combination that includes these data may be displayed as unselectable on the screen. At this point, the computing device may visually distinguish the unselectable item by various methods such as displaying it in gray or making it appear more transparent compared to other items.

Referring to the area 3b shown in FIG. 6, which displays different stages of the orthodontic treatment plan, the computing device may distinguish and display each stage by methods such as changing the font weight or color of the text "Preparation" or adding objects, allowing the user to easily recognize that the process is currently in step S 100.

Additionally, on the initial screen of step S100, the matching and separation results of the scan data necessary for orthodontic treatment may be displayed. At this time, additional data may be displayed, or some data may be modified according to the user's needs.

In this way, the lateral, frontal, and occlusal relationships that are commonly used for orthodontic treatment may all be reflected and displayed on a single screen, thereby improving user convenience. In addition, the user may select any of the areas displaying the lateral, frontal, and occlusal relationships on the screen to further review or modify details related to the selected area.

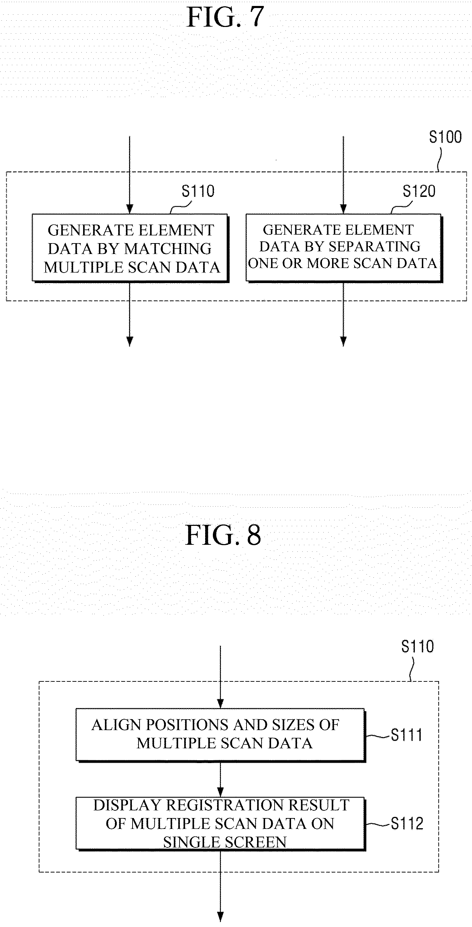

FIG. 7 is an exemplary flowchart for describing detailed processes of some operations shown in FIG. 2.

As shown in FIG. 7, step S100 may include step S110 in which the computing device matches multiple scan data, and step S120 in which the computing device separates one or more scan data. In the process of identifying a problem item for orthodontic treatment, there may be a need to match multiple scan data or separate certain scan data from one or more scan data obtained from the patient. Accordingly, the computing device may match multiple scan data among the one or more scan data obtained from the patient to generate element data. Additionally, the computing device may separate the one or more scan data obtained from the patient to generate element data.

Meanwhile, FIG. 7 illustrates that steps S110 and S120 can be performed in parallel; however, the order of the processes for steps S110 and S120 is not limited thereto. That is, steps S110 and S120 may be performed simultaneously, or step S120 may be performed after step S110 has been performed.

For example, in step S110, the computing device may match facial scan data and dental scan data, and subsequently, in step S120, the computing device may generate element data that includes information on individual teeth by separating certain data based on various types of information, such as the position information of the matched facial scan data and dental scan data.

FIG. 8 is an exemplary flowchart for describing detailed processes of some operations shown in FIG. 7.

As shown in FIG. 8, step S110 of matching multiple scan data may include step S111 in which the positions and sizes of different scan data are aligned according to preset criteria, and step S112 in which the matching result of the scan data is displayed on a single screen.

Referring to FIGS. 7 and 8, in step S110 of matching multiple scan data, the computing device may adjust the position or size information of soft tissues (facial) and hard tissues (skeletal, dental). More specifically, to ensure reproducibility of issues or predictability of treatment, the computing device may align the positions and sizes of each data. In this case, the matching reference of the scan data may be 3D data (CT), and the size or position information of each scan data may be incorporated during the scan data matching process. Additionally, the computing device may detect feature points of the data matched with the reference data (CT) during the matching of each scan data, and use the detected data to modify the size information and position information of matching data. Furthermore, the modified information may be reflected in the matching data.

Meanwhile, in step S120 of separating scan data, the computing device may detect crown information from dental data as well as root, maxillary skeletal, and mandibular skeletal information from skeletal data, and may integrate the position information of these data. Moreover, the user may review and, if necessary, modify the axes (e.g., long axis and facial axis of clinical crown (FACC)) and landmarks (e.g., proximal point, occlusal point) derived from the individual tooth information.

FIG. 9 is an example diagram illustrating a screen for matching multiple scan data in step S100 according to one embodiment of the present disclosure.

As shown in FIG. 9, the matching result of facial scan data and lateral cephalometric imaging data (cephalometric data) may be displayed, and a screen may be provided where the matching result can be easily understood without additional user operation.

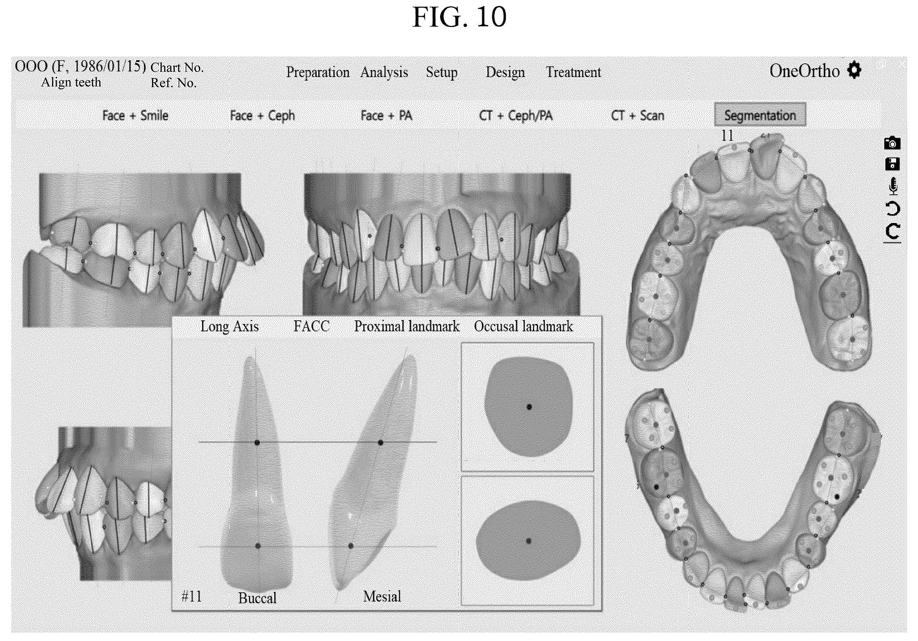

FIG. 10 is an example diagram of a screen for data separation in step S100 according to one embodiment of the present disclosure.

As shown in FIG. 10, when displaying individual tooth information, the computing device may display multiple 3D views (frontal view, rear view, left lateral view, right lateral view, maxillary occlusal plane, and mandibular occlusal plane) that allow the measurement information of the tooth and the data separation results to be understood at a glance. Furthermore, the computing device may display a screen containing the basis for modifications deemed necessary during user modification.

More specifically, when the user selects one of the teeth displayed in the multiple 3D views, the computing device may provide an interface that enables the user to make modifications by displaying a pop-up window which allows the user to check individual information of the selected tooth (e.g., Long Axis, FACC, Landmark, etc.). The user may close the pop-up window by selecting the background area that displays the teeth not included in the selection.

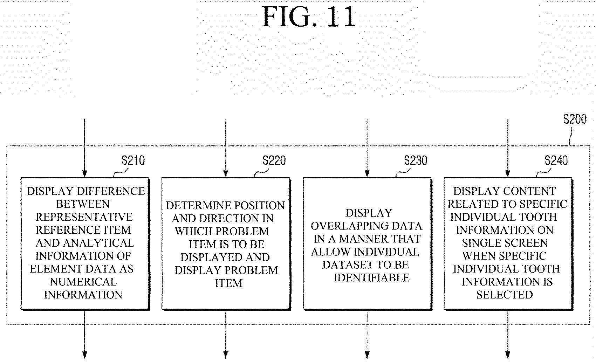

FIG. 11 is an exemplary flowchart for describing detailed processes of some operations shown in FIG. 2. Step S200 of identifying and displaying problem items for orthodontic treatment may include a plurality of steps S210, S220, S230, and S240 described below. These steps S210, S220, S230, and S240 may be performed in parallel. However, this is a mere exemplary embodiment for achieving the objectives of the present disclosure, and some steps may be added or omitted, as necessary.

As shown in FIG. 11, step S200 may include step S210 of displaying a difference between a representative reference item and analytical information of the element data as numerical information, step S220 of determining a position and a direction in which the problem item is to be displayed and displaying the problem item accordingly, step S230 of, when multiple data are overlapped, displaying the data by distinguishing each data using identifiable visual information, and step S240 of displaying content related to specific individual tooth information on a single screen when the specific individual tooth information is selected.

In step S200, the computing device may identify problem items for orthodontic treatment and display the identified problem items along with comparison results for each item. More specifically, while the initial screen (e.g., lateral, frontal, occlusal views) displayed in step S100 may maintain its layout, additional representative reference items may be displayed on the same screen. At this time, the problem items may refer to the items where differences are identified by the computing device after comparing the analytical information of the patient data with the reference numerical values for the representative reference items.

Additionally, evaluation criteria for the representative reference items may be categorized based on the patient's lateral view, frontal view, and the occlusal plane of teeth. First, for the lateral view, the representative reference items may include the esthetic line (E-line) and an anteroposterior dental relation for evaluating facial protrusion, as well as the occlusal plane for evaluating vertical relationship. For the frontal view, the representative reference items may include the dental lateral relationship midline for evaluating dental lateral relationship and facial/skeletal asymmetry, as well as the occlusal plane for evaluating facial/skeletal asymmetry. For the occlusal plane, the representative reference items may include the ideal arch (maxillary and mandibular) as criteria for evaluating tooth arrangement relationship and space deficiency.

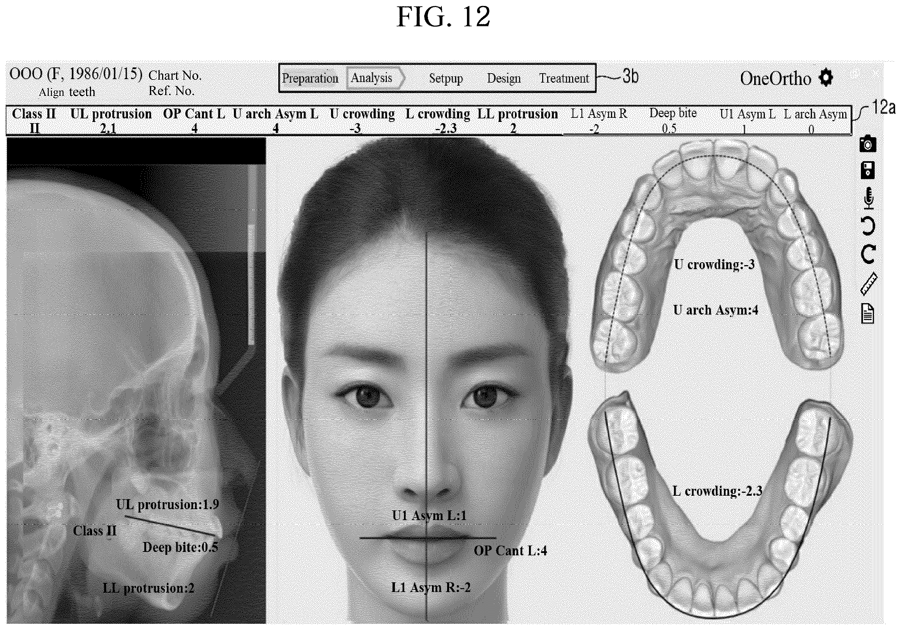

FIG. 12 is an example diagram of a problem item display screen in step S200 according to one embodiment of the present disclosure.

Referring to an area 12a shown in FIG. 12, where the representative reference items are listed, item "Class II" indicating malocclusion where the upper jaw protrudes further than the lower jaw may be displayed on the far left. In addition, the representative reference items listed after item "Class II" may be displayed in order of the greatest difference between the reference values and the analyzed values for each problem item.

Referring to the area 3b shown in FIG. 12, which displays the different stages of the orthodontic treatment plan, the computing device may distinguish and display each stage by methods such as changing the font weight and color of the text "Preparation" and "Analysis" or adding objects, allowing the user to easily recognize that step S100 has been completed and the process is currently in step S200.

As shown in FIG. 12, the problem items may be displayed in a distinguishable manner based on the degree of difference between the reference values and the analyzed values for each item. For example, if the numerical difference is significant, the computing device may display the corresponding problem item and comparison results in red or with an increased font weight.

At this time, the pertinent problem item and the comparison results may be displayed at positions that allow the user to intuitively identify the problem item. Furthermore, the user may select the displayed numerical value to view additional details of the corresponding item.

Additionally, the problem items may be displayed on individual or integrated screens for the corresponding data in a direction (e.g., frontal, rear, left lateral, right lateral, or occlusal plane) that facilitates better verification of the relevant content. Moreover, when facial, skeletal, and dental data, or tooth and gum data are displayed in an overlapping manner, levels of transparency for each data may be varied to ensure that the pertinent data is clearly distinguished, along with the direction in which it is presented.

In other words, the computing device may determine the position and direction in which the problem items are to be displayed to facilitate user comprehension and may display the problem items according to the determined position and direction. In addition, when multiple data related to the problem items are presented in an overlapping manner, the computing device may display the multiple data by distinguishing each data using identifiable visual information.

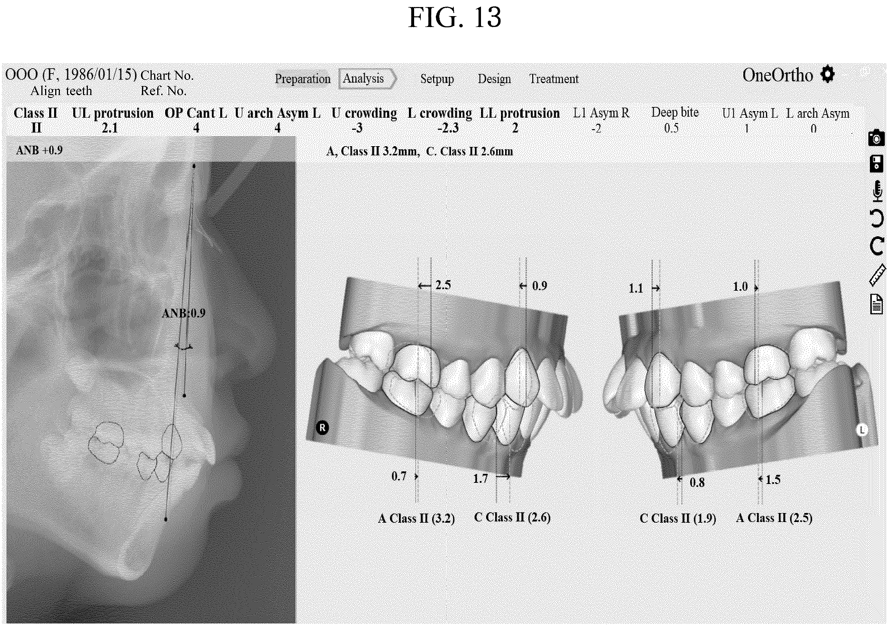

FIG. 13 is an example diagram of a detailed analysis result screen for problem items in step S200 according to one embodiment of the present disclosure.

The user may select problem items, and as shown in FIG. 13, details for each selected analysis item may be displayed. At this time, a screen that allows the user to view the identified problem items along with their associated analysis items may be displayed. More specifically, referring to FIG. 13, in response to the user's selection input of the item Class II indicating malocclusion, the computing device may display specific details serving as the basis for the classification as Class II malocclusion on the screen. Furthermore, the computing device may display the reference values for the occlusal relationship and also display both the direction and the numerical values by which the patient's current occlusal line needs to be moved to align with an occlusal line.

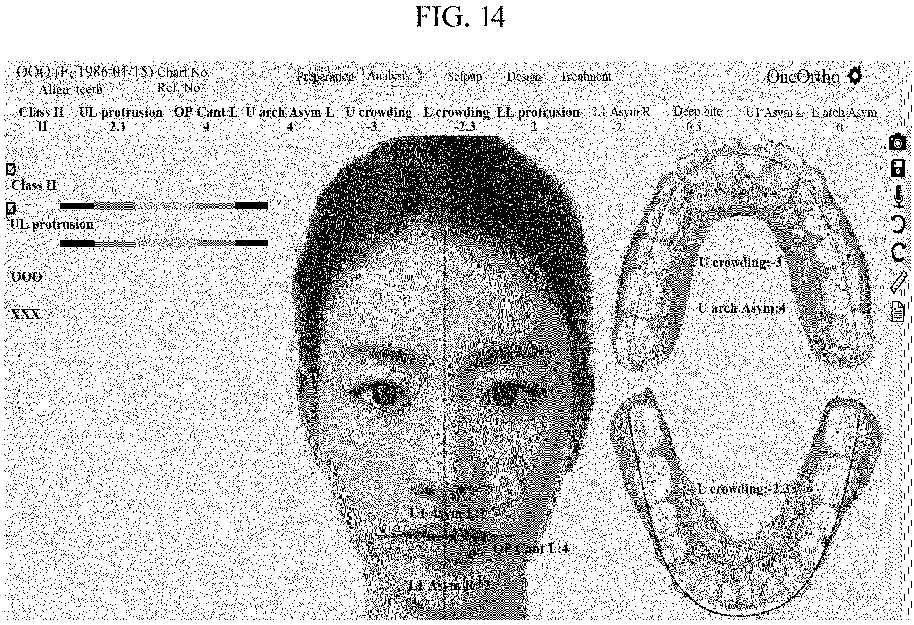

FIG. 14 is an example diagram of a screen for adjusting the range of analysis values for problem items in step S200 according to one embodiment of the present disclosure.

Referring to FIG. 14, the user may adjust the reference values and ranges for each problem item, and the computing device may determine whether to reflect these adjustments in the generation of orthodontic treatment objectives according to the selection of the corresponding items.

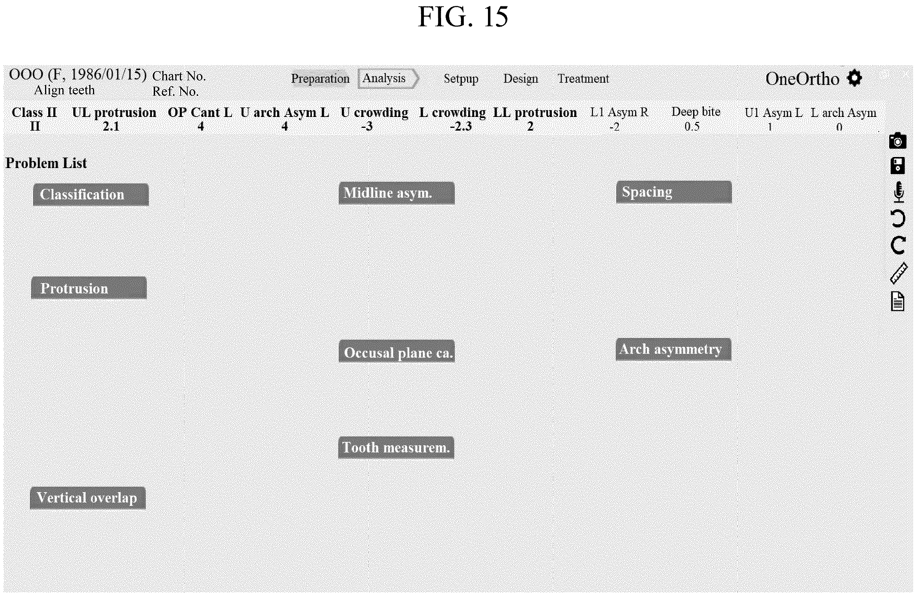

FIG. 15 is an example diagram of a screen in which the detailed analysis results for the problem items in step S200 are categorized according to one embodiment of the present disclosure.

As shown in FIG. 15, all analysis results for the identified problem items may be categorized and displayed.

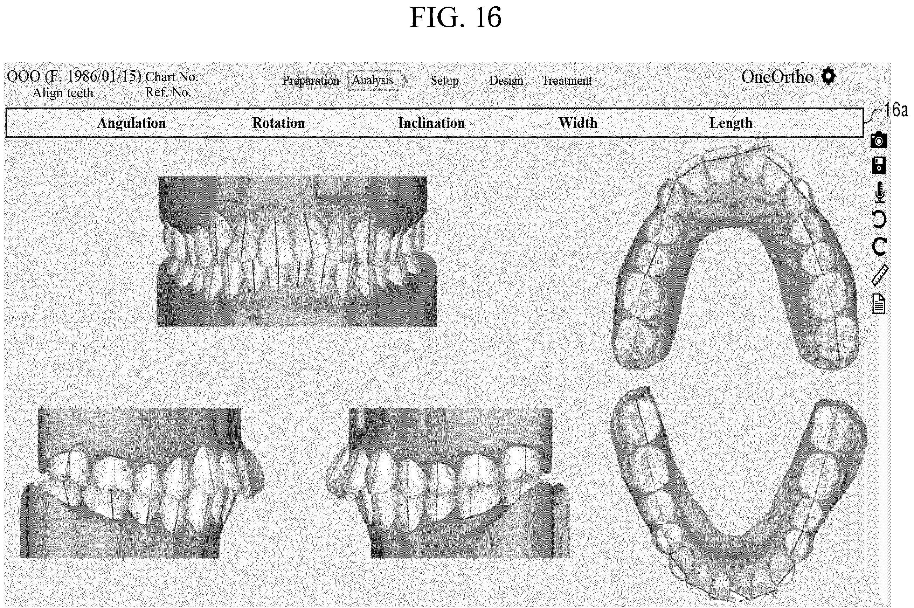

FIG. 16 is an exemplary diagram of a screen displaying individual tooth measurement information in step S200 according to one embodiment of the present disclosure.

As shown in FIG. 16, in response to the user's selection input for the area 16a listing measurement items of individual teeth, the computing device may display the complete measurement results for the selected tooth, including size (Width and Length) and angle (Angulation, Rotation, and Inclination) information. At this time, if the user selects the measurement information of an individual tooth, the computing device may display all related details on a single screen. Additionally, the computing device may display the measurement information of the individual tooth in a pop-up window or switch to a screen that displays only the selected tooth.

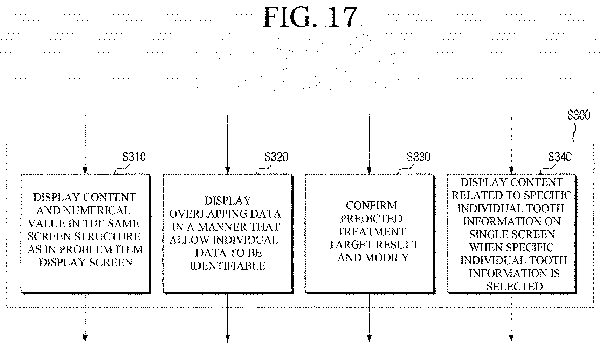

FIG. 17 is an exemplary flowchart for describing detailed processes of some operations shown in FIG. 2. Step S300, in which the computing device generates and displays an orthodontic treatment goal for the patient using the problem items identified in step S200, may include a plurality of steps S310, S320, S330, and S340 as described below. These steps S310, S320, S330 and S340 may be performed in parallel. However, this is a mere exemplary embodiment for achieving the objectives of the present disclosure, and some steps may be added or omitted, as necessary.

As shown in FIG. 17, step S300 may include step S310, in which the computing device displays the content reflecting information for the orthodontic treatment objectives, along with the analysis numerical values adjusted based on this content, in the screen structure that maintains the same layout as in the initial screen (lateral, frontal, occlusal views), step S320, in which when multiple data are overlapped, the computing device displays the multiple data by distinguishing each data using identifiable visual information, step S330, in which the computing device confirms the predicted result using the reference values of the problem items or a modified target value input by the user and allows the user to modify the orthodontic treatment objectives, and step S340, in which when individual tooth information is selected, the computing device displays the content related to the corresponding information on a single screen.

At this time, the target value in step S330 may differ from the reference value of the problem item previously set by the user. Specifically, the reference value may be modified to the target value through user input at any stage in the orthodontic treatment process, from the beginning to the end.

Meanwhile, in step S300, the computing device may generate an orthodontic treatment objective for the patient using the problem items identified in step S200. In this case, the orthodontic treatment objective may be generated in a way that reduces the deviation from the normal values of the identified problem items.

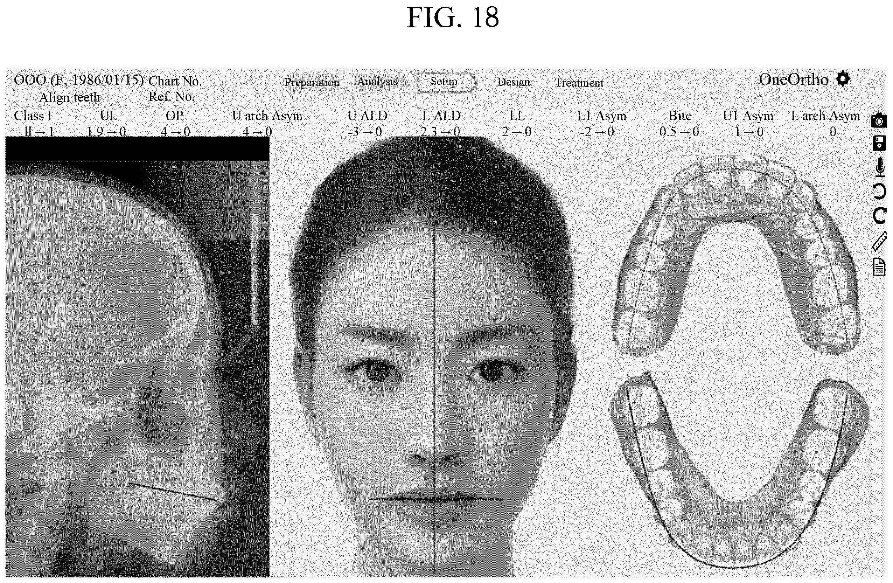

FIG. 18 is an example diagram of a screen displaying orthodontic treatment objective results in step S300 according to one embodiment of the present disclosure.

In step S300, the computing device may display an orthodontic treatment objective that can resolve the problem items identified in step S200. The computing device may display simulation results, in which the discrepancies in the problem items are adjusted to conform to the reference values, in the same screen structure as shown in step S200. In other words, the computing device may update the screen displayed in step S200 to display information for the orthodontic treatment objectives, together with analysis numerical values adjusted based on the orthodontic treatment objectives.

As shown in FIG. 18, the orthodontic treatment objective results may be displayed on individual or integrated screens of the corresponding data in a direction (e.g., frontal, rear, left lateral, right lateral, or occlusal plane) that facilitates better verification of the relevant content. Moreover, when facial, skeletal, and dental data, or tooth and gum data are displayed in an overlapping manner, levels of transparency for each data may be varied to ensure that the pertinent data is clearly distinguished, along with the direction in which it is displayed. Meanwhile, the computing device may display the orthodontic treatment objective on a screen that maintains the structure of the previous step, step S200, while incorporating all changed items.

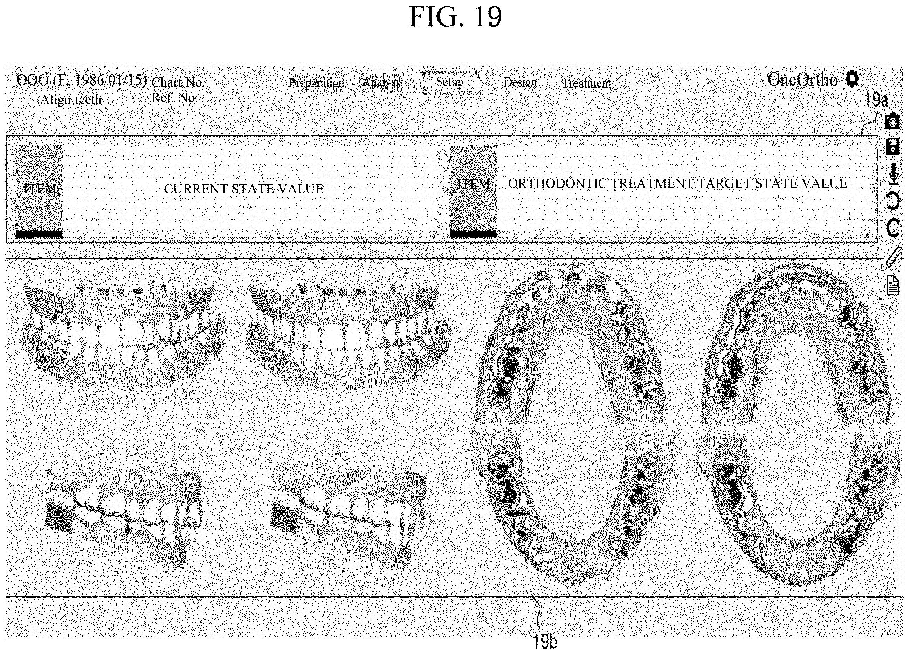

FIG. 19 is an example diagram of an orthodontic treatment objective comparison screen in step S300 according to one embodiment of the present disclosure.

As shown in FIG. 19, a comparison screen displaying the current state and the orthodontic treatment target state may be displayed. At this time, this comparison screen may be divided into an area 19a where the comparison results between the current state and the orthodontic treatment target state are displayed numerically and an area 19b where the comparison results are displayed graphically. Through this, the computing device may display the displacement of each individual tooth as a quantified result, and present both numerical values and graphics for the differences in the derived results, so that the intuitiveness for the user to understand the differences in the derived results can be enhanced.

Meanwhile, when the computing device simultaneously displays multiple sets of comparison data, the computing device may vary the transparency or color of each data in the overlapped state, ensuring that each data is visually distinguishable. Additionally, if the user selects a specific area on the full display screen, the computing device may additionally display enlarged details and allow the user to designate the gum, tooth, occlusion state, or the like as a region of interest, which may then be displayed highlighted.

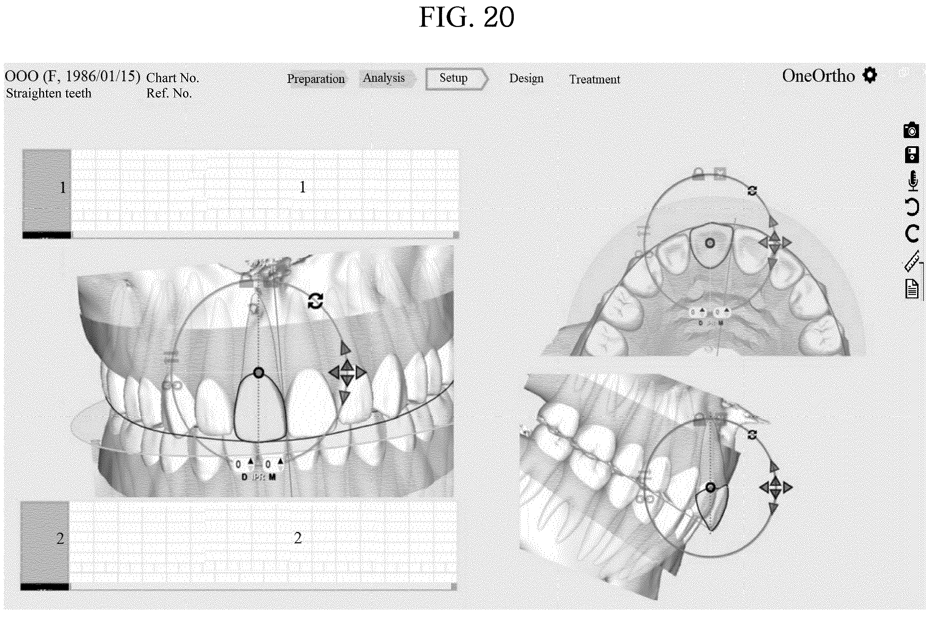

FIG. 20 is an example diagram of a screen for modifying tooth arrangement in step S300 according to one embodiment of the present disclosure.

As shown in FIG. 20, the user may modify the position and information of a selected tooth through the tooth alignment modification screen. When the user directly modifies the tooth arrangement to finalize the orthodontic treatment objectives, the computing device may display the frontal state, occlusal plane state, and lateral state simultaneously, enhancing user convenience and intuitiveness. Furthermore, matters related to the orthodontic treatment, such as numerical changes due to the modified arrangement, interproximal reduction, cross-sectional display, or addition of additional devices, may be displayed together, which may reduce the procedures and time required for the orthodontic treatment process.

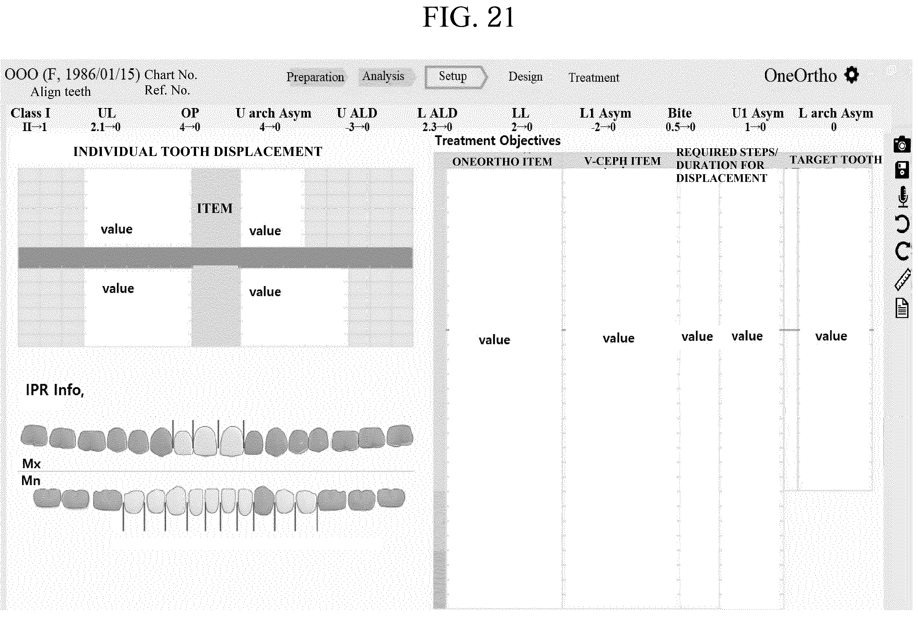

FIG. 21 is an example diagram of a summary screen of the orthodontic treatment objectives in step S300 according to one embodiment of the present disclosure.

As shown in FIG. 21, the overall results of the orthodontic treatment objectives may be categorized and displayed. At this time, the orthodontic treatment objective result screen may display quantified information, such as improvements in the differences of problem items and the target displacement (tooth movement value) for each individual tooth.

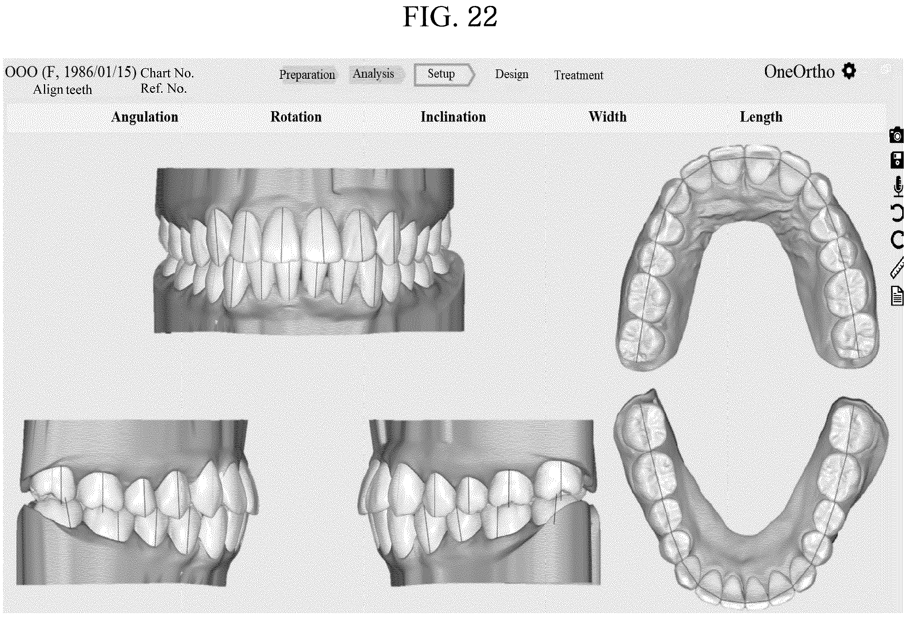

FIG. 22 is an example diagram of a screen showing individual tooth measurement for the orthodontic treatment objectives in step S300 according to one embodiment of the present disclosure.

As shown in FIG. 22, the complete information on the size (Width, Length) and angle (Angulation, Rotation, Inclination) of individual teeth for the orthodontic treatment objectives may be displayed. In this case, if the user selects the individual tooth measurement information for the orthodontic treatment objectives, the computing device may display all the relevant details on a single screen.

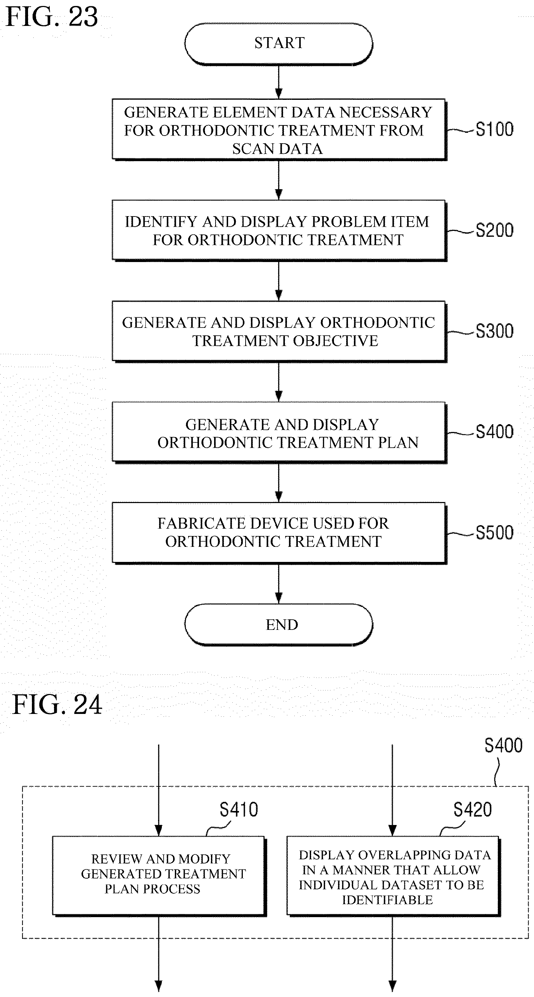

FIG. 23 is a flowchart illustrating a method for generating an orthodontic treatment plan according to another embodiment of the present disclosure.

Referring to FIG. 23, a method for generating an orthodontic treatment plan according to another embodiment of the present disclosure may further include steps S400 and S500, in addition to steps S100 to S300 shown in FIG. 2. However, this is a mere exemplary embodiment for achieving the objectives of the present disclosure, and some steps may be added or omitted, as necessary.

In step S400, the computing device may generate an orthodontic treatment plan for the patient using the information for the orthodontic treatment objectives generated in step S300 and display the information for the generated orthodontic treatment plan. More specifically, based on the generated orthodontic treatment objectives, the computing device may generate an orthodontic treatment plan that includes details such as orthodontic sequence, treatment duration, and orthodontic devices, either for each problem item or following a predefined order. At this time, the computing device may consider the interrelationships between problem items that may affect each other during the orthodontic treatment process to generate the orthodontic treatment plan.

In step S500, the computing device may output a control signal for fabricating an orthodontic device used for the orthodontic treatment of the patient, based on the orthodontic treatment plan generated in step S400.

FIG. 24 is an exemplary flowchart for describing detailed processes of some operations shown in FIG. 23. Step S400, which generates and displays the orthodontic treatment plan for the patient using the information for the orthodontic treatment objectives generated in step S300, may include a plurality of steps S410 and S420 described below. These steps S410 and S420 may be performed in parallel. However, this is a mere exemplary embodiment for achieving the objectives of the present disclosure, and some steps may be added or omitted, as necessary.

As shown in FIG. 24, step S400 may include step S410 of regenerating the orthodontic treatment plan process through a step in which the user reviews and modifies the generated orthodontic treatment plan process, and step S420 of displaying multiple data by distinguishing each data using identifiable visual information when the multiple data are displayed in an overlapping manner.

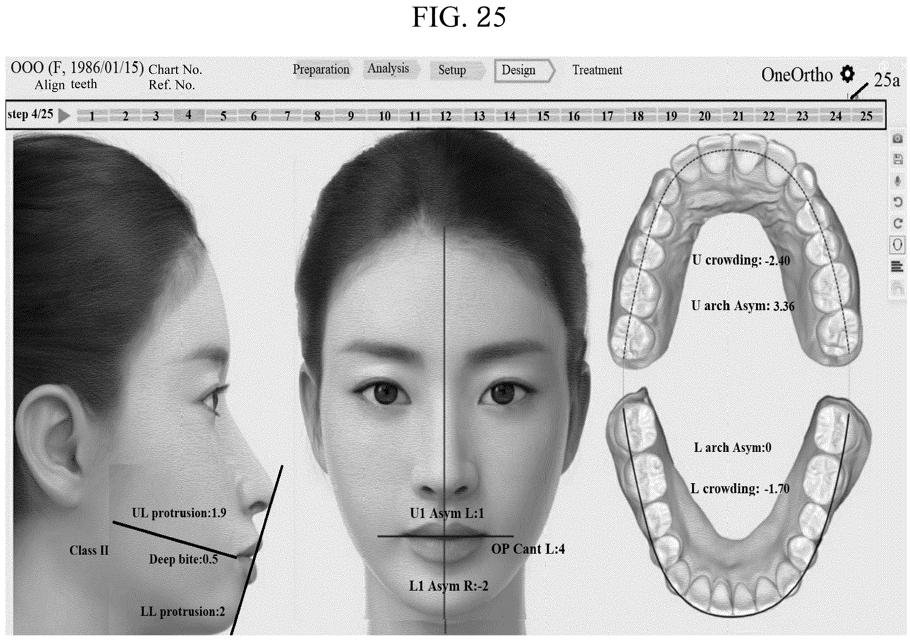

FIG. 25 is an example diagram of a screen showing the results of a treatment process in step S400 according to one embodiment of the present disclosure.

As shown in FIG. 25, an area 25a, which displays the sequential procedures of the orthodontic treatment process in a progress bar format, may be presented at the top portion of the area 3e, where related content associated with the orthodontic treatment plan process is displayed.

Through the area 25a which displays the orthodontic treatment process in a progress bar format, the user may directly select and review any state of the process, and when the user selects a continuous screen view, the computing device may display the planned treatment process step by step on the screen.

**In** step S400, the computing device may display the treatment process for addressing the problem items finalized in step S300. The computing device may divide the treatment process into steps where the problem items to be resolved according to the orthodontic treatment objective are improved and may display the simulation results for each step of the treatment process in the same screen structure as shown in step S200. In other words, predictive content reflecting the treatment process and analysis numerical values adjusted based on details reflected in each treatment step may be displayed together within the same screen structure.

Referring to FIG. 25, the treatment process screen may be displayed on individual or integrated screens of the corresponding data in a direction (e.g., frontal, rear, left lateral, right lateral, occlusal plane) that facilitates better verification of the relevant content. Moreover, when facial, skeletal, and dental data, or tooth and gum data are displayed in an overlapping manner, levels of transparency for each data may be varied to ensure that the pertinent data is clearly distinguished, along with the orientation in which the data is displayed. Furthermore, the computing device may display the treatment process, reflecting all updated details, in a screen structure consistent with the initial screen in step S300.

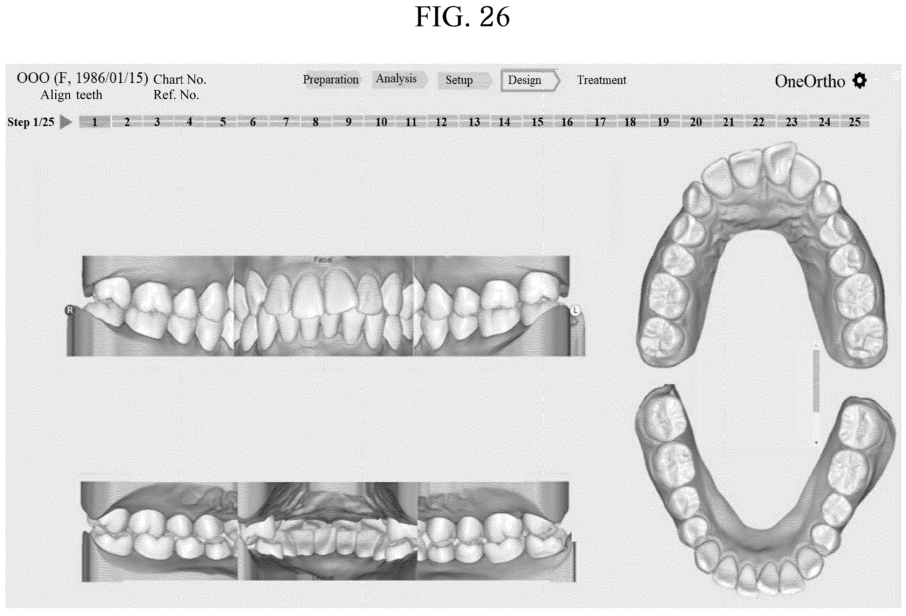

FIG. 26 is an example diagram of a screen showing step-by-step details of the treatment process in step S400 according to one embodiment of the present disclosure.

Referring to FIG. 26, the user may check detailed individual tooth movement information and specific orthodontic progress for a selected treatment step within the screen structure of frontal, rear, and occlusal plane views.

More specifically, the computing device may simultaneously display the frontal surface, which facilitates esthetic evaluation, and the rear surface, which facilitates functional evaluation, and may reconstruct and display each of the frontal and rear surfaces in a horizontal structure resembling a panoramic X-ray format which is commonly used in clinical practice. Additionally, the detailed movement amounts for individual teeth at each treatment step may be displayed above the teeth for the maxilla and below the teeth for the mandible.

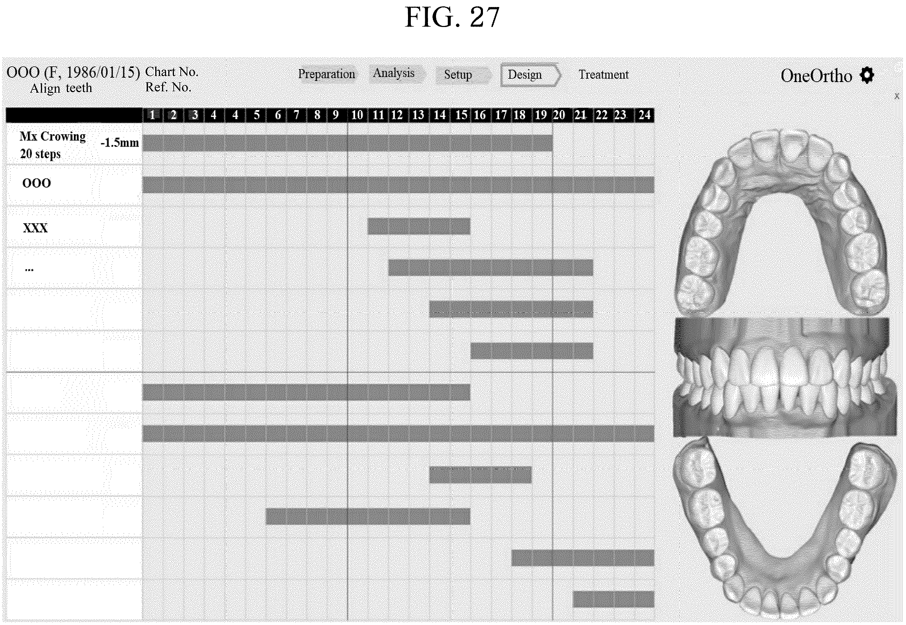

FIG. 27 is an example diagram of a screen displaying improvements in analysis items based on tooth movement changes in step S400 according to one embodiment of the present disclosure.

Referring to FIG. 27, the user may check the analysis item improvements (resolved items) according to tooth movement changes during the progression of orthodontic treatment objective.

More specifically, the computing device may display the analysis items being improved during the treatment process by associating the analysis items defined in the step preceding step S400 with individual tooth movements. At this time, the user may adjust the schedule (timing, duration) of the displayed analysis items, ultimately modifying the related movement plan for the corresponding tooth.

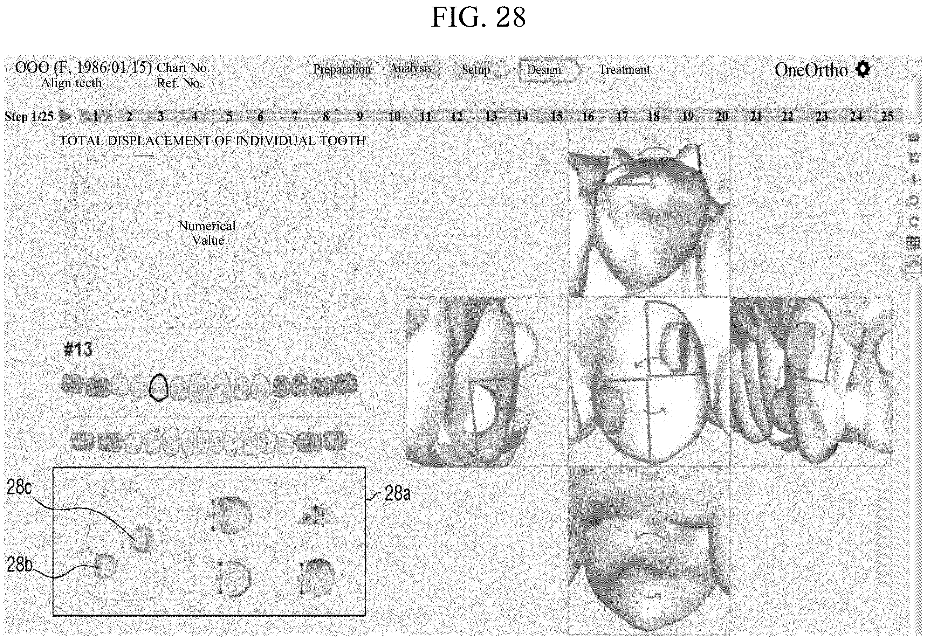

FIG. 28 is an example diagram of a screen displayed when additional fixation force is required due to tooth movement in step S400 according to one embodiment of the present disclosure.

Referring to an area 28a shown in FIG. 28, which displays detailed information about additional fixation devices, the computing device may display the corresponding additional fixation devices 28b and 28c along with the rationale for the additional fixation force deemed necessary due to tooth movement. At this time, the computing device may determine and display the size, position, and shape of the additional fixation devices based on the magnitude and direction of the required additional fixation force.

In other words, the computing device may generate the orthodontic treatment process by applying the fixation force exerted on individual teeth through the additional fixation devices during the treatment period, and it may display the relevant information on a single screen for each direction.

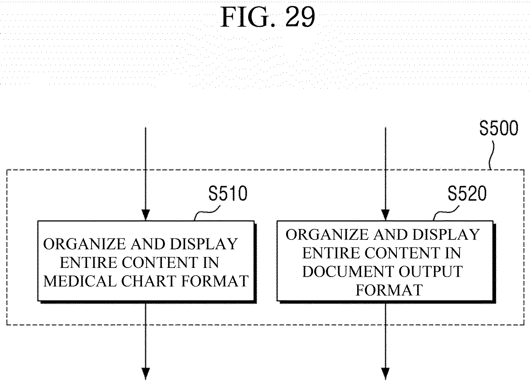

FIG. 29 is an exemplary flowchart for describing detailed processes of some operations shown in FIG. 23.

As shown in FIG. 29, step S500 of outputting a control signal for fabricating an orthodontic device used for the patient's orthodontic treatment based on the orthodontic treatment plan generated in step S400 may include step S510, in which the entire content of the orthodontic treatment plan generation process is organized in a medical chart format and displayed on a screen, and step S520, in which the entire content of the orthodontic treatment plan generation process is organized in a document output format and displayed on the screen. At this time, steps S510 and S520 may be performed in parallel. However, this is a mere exemplary embodiment for achieving the objectives of the present disclosure, and some steps may be added or omitted, as necessary.

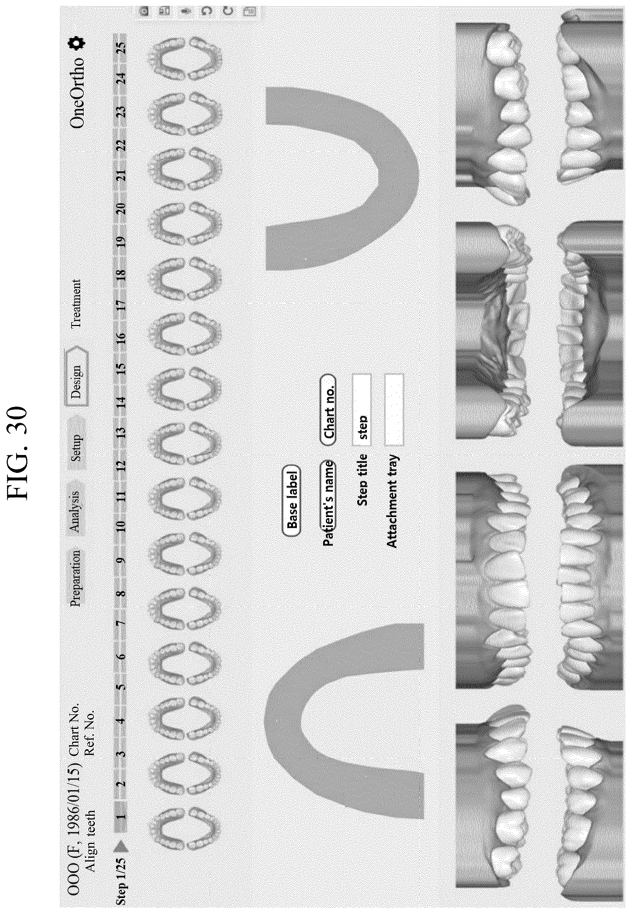

FIG. 30 is an example diagram of a screen displaying treatment device data for certain steps included in the treatment plan finalized through step S400 according to one embodiment of the present disclosure.

In the process of generating the orthodontic treatment plan through step S400, once the necessity and details of additional fixation devices are determined, the computing device may compile the information displayed during the generation of the orthodontic treatment plan and derive treatment device data that can be applied to each step of the orthodontic treatment plan. Furthermore, the computing device may use the derived treatment device data to generate a corresponding treatment device for each step of the orthodontic treatment plan.

Referring to FIG. 30, a screen may be displayed, which enables the treatment device data finalized in the step preceding step S500 to be applied to treatment through a 3D output device. More specifically, the screen illustrated in FIG. 30 may display treatment device data for the first step, selected by the user, from an orthodontic treatment plan divided into a total of 25 steps. At this time, the computing device may display identifiers (e.g., patient information, Step 1) to facilitate the differentiation of the devices being output, and if modifications are made by the user, the related details may be uniformly updated.

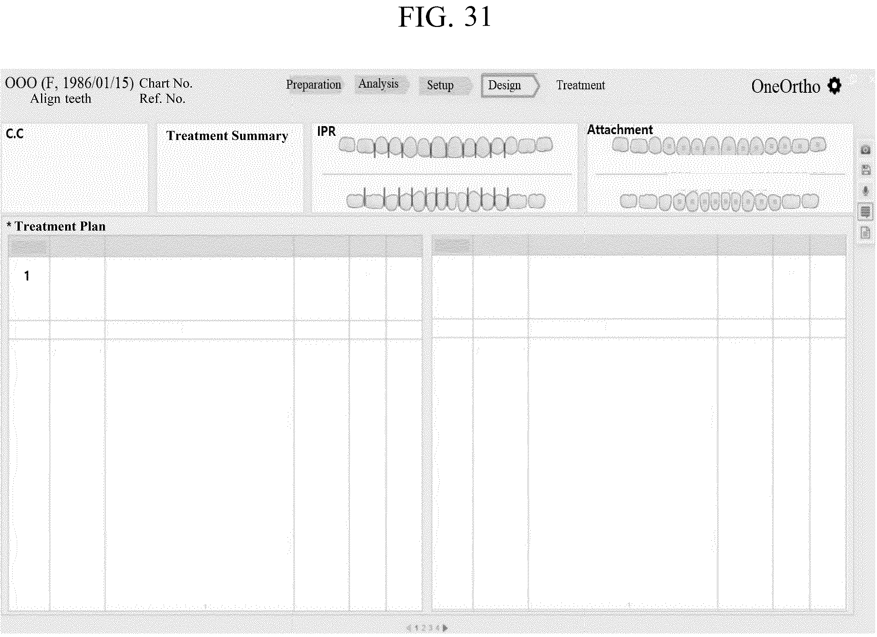

FIG. 31 is an example diagram of a screen displaying information in a format suitable for inclusion in a medical chart in step S500 according to one embodiment of the present disclosure.

Referring to FIG. 31, the analysis results, orthodontic treatment objectives, and the complete contents of the treatment plan, which can be obtained during the orthodontic treatment plan generation process, may be displayed on the screen in a format suitable for inclusion in a medical chart. At this time, the complete orthodontic treatment plan content may be linked to electronic charting functions (inside, outside, and support forms of software) of the hospital's patient management system (PMS). In other words, the computing device may transmit the orthodontic treatment plan converted into a medical chart format to the hospital's PMS, ensuring that the plan is recorded in the patient's electronic chart.

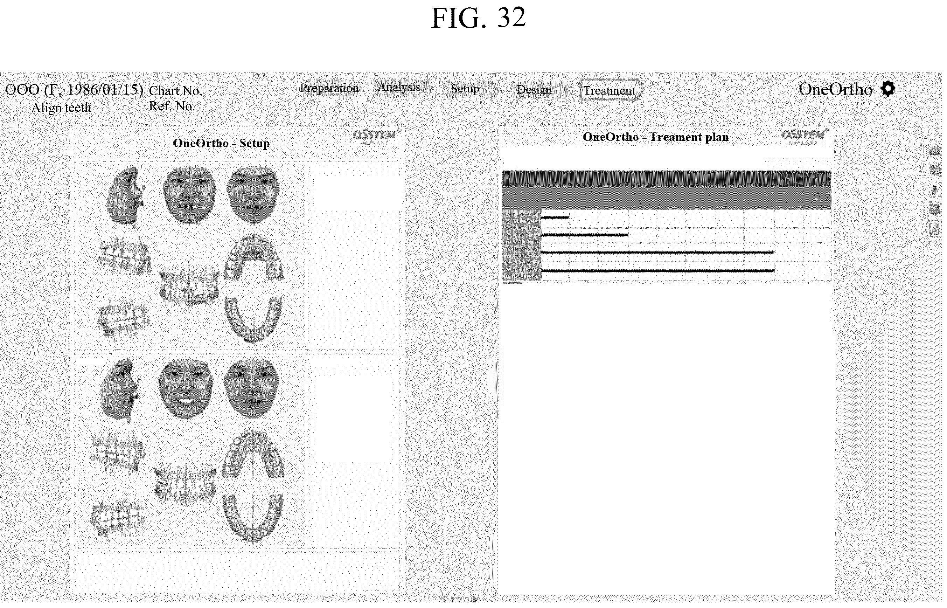

FIG. 32 is an example diagram of a screen displaying a document-printable format for step S500 according to one embodiment of the present disclosure.

Referring to FIG. 32, the analysis results, orthodontic treatment objectives, and the complete contents of the treatment plan, which can be obtained during the orthodontic treatment plan generation process, may be displayed on the screen in a format suitable for document printing. At this time, the user may modify the document output template, and the computing device may incorporate the modified details into the document output format and display it accordingly.

Meanwhile, given the long-term nature of orthodontic treatment, the computing device may reacquire scan data for the patient at user-specified intervals or points in time and repeat the entire orthodontic treatment process. At this time, the user may verify whether the treatment aligns with the orthodontic treatment objective plan and modify the goal or process as needed. The computing device may regenerate the orthodontic treatment objective plan using the modified goal and either proceed with the originally planned treatment process or execute the modified process.

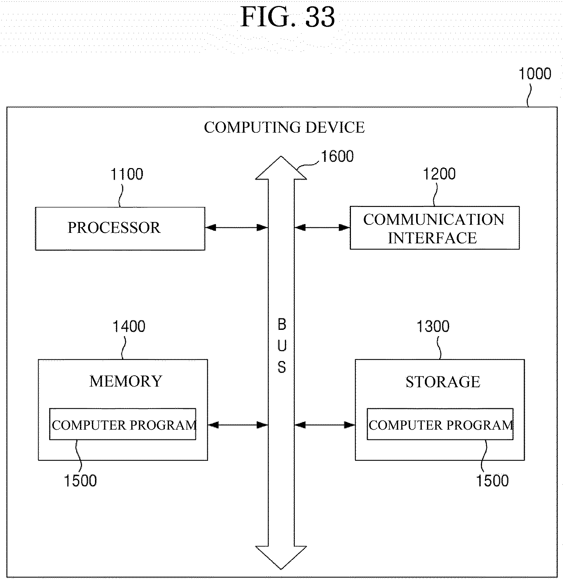

FIG. 33 is a hardware configuration diagram of a computing device according to some embodiments of the present disclosure.

FIG. 33 is a hardware configuration diagram of a computing device according to some embodiments of the present disclosure. A computing device 100 shown in FIG. 33 may include at least one processor 1100, a system bus 1600, a communication interface 1200, a memory 1400 configured to load a computer program 1500 executed by the processor 1100, and a storage 1300 configured to store the computer program 1500.

The processor 1100 controls the overall operation of each configuration of the computing device 1000. The processor 1100 may perform computations for at least one application or program for executing methods and/or operations according to various embodiments of the present disclosure. The memory 1400 stores various types of data, commands, and/or information. The memory 1400 may load one or more programs 1500 from the storage 1300 in order to execute the methods and/or operations according to various embodiments of the present disclosure. The bus 1600 provides communication functionality between the components of the computing device 1000. The communication interface 1200 supports Internet communication of the computing device 1000. The storage 1300 may non-temporarily store one or more computer programs 1500. The computer programs 1500 may include one or more instructions implementing the methods and/or operations according to various embodiments of the present disclosure. When the computer program 1500 is loaded on the memory 1400, the processor 1100 may execute the one or more instructions to perform the methods and/or operations according to various embodiments of the present disclosure.

So far, various embodiments and effects thereof, in which the technical features of the present disclosure have been implemented, have been described with reference to FIGS. 1 to 33. Effects according to the technical features of the present disclosure are not limited to the above-described effects, and other effects not described will be clearly understood by those skilled in the art from the following description.

The technical features of the present disclosure described above may be implemented as computer readable codes on a computer-readable medium. The computer program recorded on the computer-readable recording medium may be transmitted to another computing device through a network such as the Internet and installed in the other computing device, thereby being used in the other computing device.

Although the operations are shown in a specific order in the drawings, it should not be understood that the operations must be performed in the specific order or sequential order shown, or that all the illustrated operations must be executed to achieve the desired results. In certain circumstances, multitasking and parallel processing may be advantageous. In concluding the detailed description, those skilled in the art will appreciate that many variations and modifications can be made to the exemplary embodiments without substantially departing from the principles of the present disclosure. Therefore, the disclosed exemplary embodiments of the invention are used in a generic and descriptive sense only and not for purposes of limitation. The scope of the present disclosure should be interpreted by the claims below, and any technical features within the scope equivalent to the claims should be interpreted as falling within the scope of the technical features defined by the present disclosure.

## Claims

1. A method for generating an orthodontic treatment plan, the method being performed by a computing device and comprising:
analyzing one or more scan data for a patient and generating element data necessary for orthodontic treatment from the one or more scan data by using the analysis result;
identifying a problem item for the orthodontic treatment of the patient using the element data and displaying the identified problem items; and
generating an orthodontic treatment objective for the patient using the identified problem item and displaying information for the generated orthodontic treatment objective.

2. The method of claim 1, wherein the scan data is data acquired using an intraoral scanner or skeletal computed tomography (CT), and
the element data is data obtained by preprocessing the scan data into a format required for identifying the problem item.

3. The method of claim 1, wherein the generating of the element data comprises generating element data by matching multiple scan data among the one or more scan data.

4. The method of claim 1, wherein the generating of the element data comprises generating the element data by separating the one or more scan data.

5. The method of claim 1, wherein the displaying of the problem item comprises determining a position and a direction in which the problem item is to be displayed; and
displaying the problem item according to the determined position and direction.

6. The method of claim 1, wherein the displaying of the problem item comprises, when multiple data related to the problem item are displayed in an overlapping manner, displaying the data by distinguishing each data using identifiable visual information.

7. The method of claim 1, wherein the displaying of the information for the orthodontic treatment objective comprises generating the orthodontic treatment objective for the patient using the identified problem item,
wherein the orthodontic treatment objective is generated in a manner that reduces deviation from a reference value of the identified problem item.

8. The method of claim 1, wherein the displaying of the information for the orthodontic treatment objective comprises updating a screen displaying the identified problem item to display the information for the orthodontic treatment objective, along with an analysis numerical value adjusted based on the orthodontic treatment objective.

9. The method of claim 1, wherein the displaying of the information for the orthodontic treatment objective comprises, when multiple data related to the information for the orthodontic treatment objective are displayed in an overlapping manner, displaying the data by distinguishing each data using identifiable visual information.

10. The method of claim 1, further comprising:
generating an orthodontic treatment plan for the patient using the information for the generated orthodontic treatment objective and displaying the generated orthodontic treatment plan; and
outputting a control signal for fabricating an orthodontic device used for orthodontic treatment of the patient, based on the generated orthodontic treatment plan.

11. The method of claim 10, wherein the outputting of the control signal comprises:
converting the orthodontic treatment plan into a medical chart format;
displaying the orthodontic treatment plan converted into the medical chart format; and
transmitting the orthodontic treatment plan converted into the medical chart format to a hospital's patient management system (PMS) so that the converted orthodontic treatment plan is recorded in an electronic chart of the patient.

12. A device for generating an orthodontic treatment plan, the device being configured as a computing device comprising a processor, and a memory,
wherein the memory is configured to store instructions, and
the instructions, when executed by the processor, cause the processor to perform the steps of:
analyzing one or more scan data for a patient and generating element data necessary for orthodontic treatment from the one or more scan data by using the analysis result;
identifying problem items for the orthodontic treatment of the patient using the element data and displaying the identified problem items; and
generating an orthodontic treatment objective for the patient using the identified problem items and displaying information for the generated orthodontic treatment objective.

13. The device of claim 12, further comprising:
generating an orthodontic treatment plan for the patient using the information for the generated orthodontic treatment objective and displaying the generated orthodontic treatment plan; and
outputting a control signal for fabricating an orthodontic device used for orthodontic treatment of the patient, based on the generated orthodontic treatment plan.
